(19) **Europäisches Patentamt / European Patent Office / Office européen des brevets**

(11) **EP 4 659 741 A1**

(12) **EUROPEAN PATENT APPLICATION**
published in accordance with Art. 153(4) EPC

(43) Date of publication:
**10.12.2025 Bulletin 2025/50**

(21) Application number: **24749669.8**

(22) Date of filing: **30.01.2024**

(51) International Patent Classification (IPC):
*A61K 9/127* (2025.01)    *A61K 31/337* (2006.01)
*A61K 31/7068* (2006.01)    *A61K 33/24* (2019.01)
*A61K 31/282* (2006.01)    *A61P 35/00* (2006.01)

(52) Cooperative Patent Classification (CPC):
A61K 9/127; A61K 31/282; A61K 31/337;
A61K 31/7068; A61K 33/24; A61P 35/00

(86) International application number:
**PCT/CN2024/074604**

(87) International publication number:
**WO 2024/160190 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.01.2023 CN 202310046953**

(71) Applicant: **CSPC Zhongqi Pharmaceutical Technology (Shijiazhuang) Co., Ltd.**
**Shijiazhuang, Hebei 050035 (CN)**

(72) Inventors:
• **LI, Chunlei**
 **Shijiazhuang, Hebei 050035 (CN)**
• **LI, Chenxiao**
 **Shijiazhuang, Hebei 050035 (CN)**

• **ZHANG, Xinna**
 **Shijiazhuang, Hebei 050035 (CN)**
• **ZHAO, Lu**
 **Shijiazhuang, Hebei 050035 (CN)**
• **ZHANG, Yi**
 **Shijiazhuang, Hebei 050035 (CN)**
• **ZHOU, Rong**
 **Shijiazhuang, Hebei 050035 (CN)**
• **NIU, Qiuyun**
 **Shijiazhuang, Hebei 050035 (CN)**
• **JIN, Chan**
 **Shijiazhuang, Hebei 050035 (CN)**
• **NIU, Yameng**
 **Shijiazhuang, Hebei 050035 (CN)**
• **LI, Chunna**
 **Shijiazhuang, Hebei 050035 (CN)**

(74) Representative: **Colombo, Stefano Paolo et al**
**Marchi & Partners S.r.l.**
**Via Vittor Pisani, 13**
**20124 Milano (IT)**

(54) **USE OF PACLITAXEL CATIONIC LIPOSOME IN TREATING TUMORS**

(57) The present invention provides a use of a paclitaxel cationic liposome in preparation of a drug for treating advanced solid tumors, a use of the paclitaxel cationic liposome and a system therapeutic drug in preparation of a drug for treating advanced solid tumors, and a method for treating advanced solid tumors. A therapeutically effective amount of paclitaxel cationic liposome is administered to patients with advanced solid tumors, or a therapeutically effective amount of paclitaxel cationic liposome and system therapeutic drug is administered to patients with advanced solid tumors.

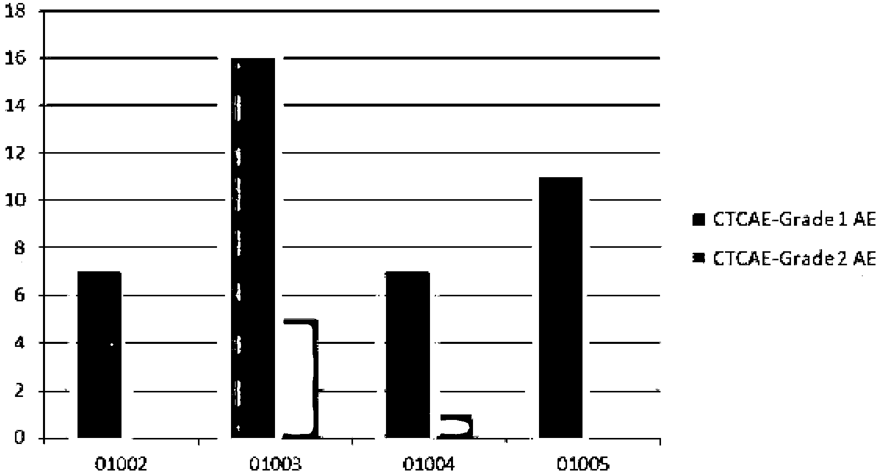

FIG. 1

**Description**

**CROSS-REFERENCE TO RELATED APPLICATION**

The present application claims the benefit of a priority of Chinese Patent Application No. 202310046953.0, filed on January 31, 2023, the entire contents of which are incorporated herein by reference.

**TECHNICAL FIELD**

The present disclosure belongs to the antitumor field, and specifically relates to a regimen of arterial infusion of paclitaxel cationic liposome in combination with systemic therapy, a regimen of arterial infusion of paclitaxel cationic liposome alone, and use in the preparation of a medicament for treating tumors, in particular advanced solid tumors, such as gastric cancer, pancreatic cancer, and colorectal cancer liver metastases.

**BACKGROUND**

Paclitaxel is a tricyclic diterpenoid, which was firstly isolated from the bark of *Taxus brevifolia* by Wani *et al.* who also confirmed the chemical structure of paclitaxel. Paclitaxel is an anti-microtubule drug capable of inducing and promoting microtubule assembly and having the functions of polymerizing with and stabilizing microtubules, which interfere with rearrangement of microtubules and result in cessation of mitosis, thereby inhibiting tumor cell growth.

Cationic liposome is a novel form of drug delivery. Cationic paclitaxel drugs have not yet been available worldwide. A similar drug, Endo TAG-1, is now at the clinical research stage.

Gastric cancer refers to a malignant tumor of epithelial origin that originates in the stomach. According to the latest data from China in 2020, both the incidence and mortality of gastric cancer rank third among all kinds of malignant tumors. There are about 1.2 million new cases of gastric cancer worldwide per year, about 40% of which occurs in China. Early gastric cancer in China takes up a very low proportion, only about 20%. Most of gastric cancers have already been in the progressive stage when they are diagnosed, with an overall 5-year survival rate of less than 50%.

The incidence and mortality of colorectal cancer in China rank third and fifth respectively among all the malignant tumors, with 376,000 new cases and 191,000 deaths. Among them, the incidence and mortality in urban areas are much higher than those in rural areas, and the incidence of colon cancer has increased significantly. Most of the patients were diagnosed at advanced stage, and the 5-year survival rate of advanced rectal cancer is less than 10%.

Statistics from the National Central Cancer of China in 2021 show that pancreatic cancer ranks 7th in male and 11th in female in the incidence of malignant tumors, and accounts for the 6th highest malignant tumor-associated mortality.

Ovarian cancer, as one of the common malignant tumors in women, is mostly found in the advanced stage due to lack of typical symptoms and signs, and the survival period is very short. In 2020, there were 55,342 new cases of ovarian cancer and 37,519 new deaths in China. Ovarian cancer ranks third in terms of the incidence and second in terms of the mortality among malignant tumors of the female reproductive system.

Cholangiocarcinoma is a common malignant tumor of the bile ducts, accounting for about 15-20% of all primary hepatobiliary cancers. It occurs mostly in people older than age 50. Its 5-year survival rate is also less than 25%.

Prostate cancer is one of the most common malignant tumors in the male reproductive system, and its incidence ranks third following only breast cancer and lung cancer; the death cases make up 3.8% of systemic malignant tumors and the mortality ranks 8th. In 2015, prostate cancer ranks sixth in terms of the incidence and tenth in terms of the mortality among male malignant tumors in China.

Lung cancer is the malignant tumor with the fastest-growing incidence in China over the past 30 years. In 2015, there are 630,000 lung cancer deaths in China, including 433,000 men and 197,000 women, which account for 27.0% of all deaths from malignant tumors. The 5-year survival rate of lung cancer is estimated to be only 8.9% in Europe, China, and developing countries.

Primary liver cancer is currently the fourth most common malignant tumor and the second leading cause of tumor-related death in China, seriously threatening the lives and health of Chinese people. According to the SEER database of the National Cancer Institute, the average 5-year survival rate of HCC patients in the United States is 19.6%, but the average 5-year survival rate of advanced metastatic diseases can be as low as 2.5%.

The so-called "systemic therapy" or referred to as generalized therapy refers to treatment methods conducted mainly via oral, intramuscular or intravenous administration, including molecularly targeted therapy, immunotherapy, chemotherapy, traditional Chinese medicine therapy, etc. It includes, but is not limited to, for example, tumor treatment regimens recommended by CSCO, e.g., the XELOX regimen (also referred to as the CAPEOX regimen, capecitabine + oxaliplatin) which is a first-line treatment regimen for advanced gastric cancer, colorectal cancer, and pancreatic cancer. The research results show that after the first-line treatment with the XELOX regimen, the median PFS of patients with colorectal cancer is 8.0 months and the median OS is 19.8 months. The FOLFOX regimen (5-fluorouracil, Leucovorin calcium, and oxaliplatin) can be used for systemic chemotherapy for cholangiocarcinoma; platinums and fluorouracils can be used for systemic chemotherapy for esophageal cancer; carboplatin and gemcitabine can be used for systemic chemotherapy for ovarian cancer; sorafenib can be used for systemic chemotherapy for hepatocellular carcinoma; and PD-1 inhibitor can be used for systemic therapy for lung cancer.

For gastrointestinal tumors, the most common site of metastasis in the progression stage is the liver. Systemic chemotherapy tends to be ineffective for liver metastases. Due to the special anatomy of the liver, for patients with inoperable primary hepatocellular carcinoma, interventional modalities such as transcatheter arterial chemoembolization (TACE) and hepatic arterial infusion chemotherapy (HAIC) are often used for local treatment, which has been recommended by the CSCO for their remarkable efficacy, and this technology is relatively mature. The Guidelines of the National Health Commission of China for gastric cancer, colorectal cancer, and pancreatic cancer also recommend the interventional therapy including HAIC for these tumors for liver metastases.

Arterial infusion allows several effective chemotherapeutics to be combined together, finds the blood supply artery of the tumor through the catheter technology, and directly injects anticancer drugs into the tumor tissue or tumor bed to exert the "first-pass effect" of drugs, so as to significantly increase the local drug concentration of the tumor. Meanwhile, after arterial infusion, chemotherapeutics will also circulate throughout the whole body through the blood, so it also plays a systemic chemotherapy effect to some degree. Several clinical studies have proven the hepatic arterial infusion chemotherapy to be effective. The CSCO Guidelines for Liver Cancer (2022) include HAIC as a later line therapy for locally advanced hepatocellular carcinoma (HCC). The mode of administration by arterial infusion is expected to be effective in patients with liver metastases, improve their quality of life, and prolong their survival period.

Citation List

Non-Patent Literature 1: Chinese tumor intervention expert consensus on the application principles of transcatheter arterial infusion chemotherapy. J Intervent Radiol 2017-11, Vol.26, No.11

Non-Patent Literature 2: Cassidy J, Clarke S, Díaz-Rubio E, Scheithauer W, Figer A, Wong R, Koski S, Lichinitser M, Yang TS, Rivera F, Couture F, Sirzén F, Saltz L. Randomized phase III study of capecitabine plus oxaliplatin compared with fluorouracil/folinic acid plus oxaliplatin as first-line therapy for metastatic colorectal cancer. J Clin Oncol. 2008 Apr 20; 26(12):2006-12.

Non-Patent Literature 3: S. Strieth, M.E. Eichhorn, B. Sauer, et al. Neovasculartargeting chemotherapy: encapsulation of paclitaxel in cationic liposomes impairs functional tumor microvasculature. Int. J. Cancer, 110:117-124 (2004)

Non-Patent Literature 4: Ma WW, Hidalgo M. The winning formulation: the development of paclitaxel in pancreatic cancer. Clin Cancer Res. 2013 Oct 15; 19(20):5572-9. doi: 10.1158/1078-0432.CCR-13-1356. Epub 2013 Aug 5. PMID: 23918602.

Non-Patent Literature 5: Camacho LH, Kurzrock R, Cheung A, Barber DF, Gupta S, Madoff DC, Wallace MJ, Kim EE, Curley SA, Hortobagyi GN, Mavligit G. Pilot study of regional, hepatic intra-arterial paclitaxel in patients with breast carcinoma metastatic to the liver. Cancer. 2007 Jun 1; 109(11):2190-6.

## SUMMARY

The present disclosure provides use of a paclitaxel cationic liposome in preparation of a medicament for treating advanced solid tumors.

The present disclosure further provides use of a paclitaxel cationic liposome and systemic therapeutic drug in preparation of a medicament for treating advanced solid tumors, preferably the systemic therapeutic drug being capecitabine and oxaliplatin, or cisplatin, or gemcitabine, or capecitabine.

The present disclosure further provides use of a paclitaxel cationic liposome in preparation of a medicament for improving the efficacy of systemic therapeutic drug on advanced solid tumors.

The present disclosure provides a method for treating advanced solid tumors, wherein a therapeutically effective amount of a paclitaxel cationic liposome is administered to a patient with advanced solid tumors.

The present disclosure provides a method for treating advanced solid tumors, wherein a therapeutically effective amount of a paclitaxel cationic liposome and a systemic therapeutic drug are administered to a patient with advanced solid tumors, preferably a therapeutically effective amount of the paclitaxel cationic liposome, capecitabine, and oxaliplatin are administered to the patients with the advanced solid tumors, or preferably the paclitaxel cationic liposome is administered via arterial infusion.

The present disclosure further provides a method for improving the efficacy of asystemic therapeutic drug on advanced solid tumors, wherein a therapeutically effective amount of a paclitaxel cationic liposome is further administered to a patient in addition to the systemic therapeutic drug, preferably the systemic therapeutic drug is capecitabine and oxaliplatin, or cisplatin, or gemcitabine, or capecitabine.

In some embodiments, the advanced solid tumor described above includes, but is not limited to, gastrointestinal tumors (gastric cancer, esophageal cancer, pancreatic cancer, colorectal cancer, cholangiocarcinoma, and liver cancer), lung cancer, gynecological tumors (ovarian cancer, endometrial cancer, and cervical cancer), prostate cancer, bladder cancer, and solid tumor with liver metastases.

In some embodiments, the advanced solid tumor comprises gastric cancer, pancreatic cancer, and colorectal cancer with liver metastases.

In some embodiments, the advanced solid tumor comprises treatment-naive liver metastases, including but not limited to, gastric cancer with liver metastases, colorectal cancer with liver metastases, and pancreatic cancer with liver metastases.

The above-mentioned "systemic therapy" includes, but is not limited to, e.g. anti-tumor therapy regimens recommended by CSCO, for example, the XELOX regimen (also referred to as the CAPEOX regimen, capecitabine + oxaliplatin) which is a first-line therapy regimen for advanced gastric cancer, colorectal cancer, and pancreatic cancer. The FOLFOX regimen (5-fluorouracil, Leucovorin calcium, oxaliplatin) can be used for systemic chemotherapy for cholangiocarcinoma; platinum- and fluorouracil-based drugs can be used for systemic chemotherapy for esophageal cancer; carboplatin and gemcitabine can be used for systemic chemotherapy for ovarian cancer; sorafenib can be used for systemic chemotherapy for hepatocellular carcinoma; and PD-1 inhibitor can be used for systemic therapy for lung cancer.

The present disclosure further provides a method for improving an efficacy of a combination of capecitabine and oxaliplatin on an advanced solid tumor, for example, advanced gastric cancer, colorectal cancer or pancreatic cancer, wherein a therapeutically effective amount of a paclitaxel cationic liposome is further administered to a patient in addition to capecitabine and oxaliplatin.

The present disclosure further provides a method for improving the efficacy of a platinum- or fluorouracil-based drug on esophageal cancer, wherein a therapeutically effective amount of a paclitaxel cationic liposome is further administered to patients in addition to the platinum- or fluorouracil-based drug.

The present disclosure further provides a method for improving the efficacy of carboplatin or gemcitabine on ovarian cancer, wherein a therapeutically effective amount of a paclitaxel cationic liposome is further administered to patients in addition to carboplatin or gemcitabine.

The present disclosure further provides a method for improving the efficacy of sorafenib on hepatocellular carcinoma, wherein a therapeutically effective amount of a paclitaxel cationic liposome is further administered to patients in addition to sorafenib.

The present disclosure further provides a method for improving the efficacy of PD-1 inhibitor on lung cancer, wherein a therapeutically effective amount of a paclitaxel cationic liposome is further administered to patients in addition to the PD-1 inhibitor.

Preferably, the paclitaxel cationic liposome according to the present disclosure is present in the form of a powder for injection or a liquid injection. When the paclitaxel cationic liposome is a liquid injection, it contains, on a paclitaxel basis, 0.05 to 1 mg/ml, preferably 0.2 to 0.3 mg/ml, more preferably 0.25 mg/ml of an active ingredient.

Preferably, the therapeutically effective amount of the paclitaxel cationic liposome, on a paclitaxel basis, refers to 5 to 100 mg/m$^2$, preferably 5 to 80 mg/m$^2$, further preferably 11 to 55 mg/m$^2$, or yet further preferably 24 to 70 mg/m$^2$, or any value within the range, for example, 11 mg/m$^2$, 22 mg/m$^2$, 24 mg/m$^2$, 33 mg/m$^2$, 36 mg/m$^2$, 44 mg/m$^2$, 48 mg/m$^2$, 55 mg/m$^2$, 60 mg/m$^2$, or 70 mg/m$^2$. Preferably, the paclitaxel cationic liposome is administered via arterial infusion. Preferably, the dosing cycle is once every 3 weeks.

In some embodiments, the paclitaxel cationic liposome according to the present disclosure contains: paclitaxel, 1,2-dioleoyl-sn-glycero-3-phosphocholine (DOPC), 1,2-dioleoyl-3-trimethylammonium-propane chloride (DOTAP), and trehalose.

Preferably, the paclitaxel cationic liposome contains 5 to 10 mg of paclitaxel, 72 to 144 mg of DOPC, 68 to 136 mg of DOTAP, and 1500 to 2500 mg of trehalose in each preparation (or preparation unit).

Most preferably, each preparation (unit of preparation) contains 10 mg of paclitaxel, 144 mg of DOPC, 136 mg of DOTAP, and 2500 mg of trehalose, or each preparation (unit of preparation) contains 5 mg of paclitaxel, 72 mg of DOPC, 68 mg of DOTAP, and 2000 mg of trehalose, or each preparation (unit of preparation) contains 5 mg of paclitaxel, 72 mg of DOPC, 68 mg of DOTAP, and 1500 mg of trehalose.

The paclitaxel cationic liposome according to the present disclosure may be prepared by a conventional method in the art, or may be a paclitaxel cationic liposome prepared by any one of methods disclosed in the prior art, e.g., prepared according to the prescription and method described in US7794747B.

All the dosages of the paclitaxel cationic liposome according to the present disclosure are on a paclitaxel basis.

Advantageous Effects of the Invention

The animal experiments in the present disclosure prove that the paclitaxel cationic liposome used in combination with other drugs or treatment methods, for example, in combination with cisplatin, or gemcitabine or capecitabine, can significantly improve the antitumor efficacy, and achieve a remarkably better effect than that of the paclitaxel injection Taxol at the same dose.

Co-administration of the paclitaxel cationic liposome via arterial infusion and the XELOX regimen to patients with gastric cancer, pancreatic cancer or colorectal cancer with liver metastases could improve the efficacy of the XELOX regimen on the patients with gastric cancer, pancreatic cancer or colorectal cancer with liver metastases, increase the disease response rate, in particular the complete response rate (CR rate) and the partial response rate (PR rate), and control the progression of diseases, and the paclitaxel cationic liposome is safe and tolerable with few toxic side effects.

Administration of the paclitaxel cationic liposome or the paclitaxel cationic liposome in combination with systemic therapeutic drug(s) to patients with advanced solid tumor could improve the efficacy of the systemic therapeutic drug(s) on the patients with advanced solid tumor, increase the disease response rate, and control the progression of diseases, and the paclitaxel cationic liposome is safe and tolerable with few toxic side effects.

## BRIEF DESCRIPTION OF THE DRAWINGS

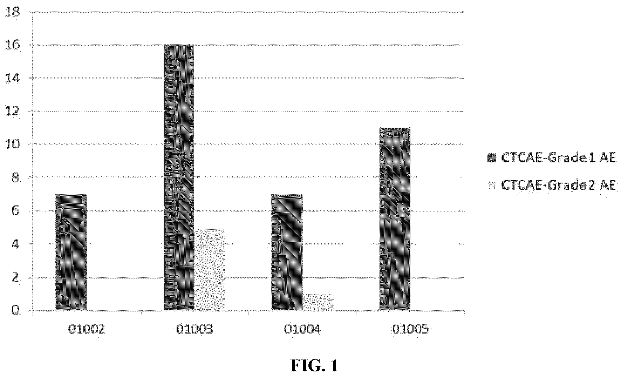

FIG. 1 shows the incidence of all-grade drug-related AE gradesfor the treatment regimen in Example 5.

## DETAILED DESCRIPTION

The examples described below are specific descriptions of the present disclosure and should not be construed to limiting the scope of the present disclosure.

### Example 1: Preparation of Paclitaxel Cationic Liposomes

The prescriptions of the paclitaxel cationic liposomes were as follows:

| Ingredients | 1-1 | 1-2 | 1-3 |
|---|---|---|---|
| Paclitaxel | 10 mg | 5 mg | 5 mg |
| 1,2-Dioleoyl-sn-glycero-3-phosphocholine (DOPC) | 144.2 mg | 72.1 mg | 72.1 mg |
| 1,2-Dioleoyl-3-trimethylammonium-propane chloride (DOTAP) | 136.4 mg | 68.2 mg | 68.2 mg |
| Trehalose | 2500 mg | 2000 mg | 1500 mg |

The preparation method was as follows:

(1) paclitaxel, DOPC, and DOTAP were weighed according to the prescribed doses, and dissolved in anhydrous ethanol to obtain an organic phase after dissolution;
(2) trehalose was dissolved in water for injection to afford 10% to 12.5% trehalose solution as an aqueous phase;
(3) the oil phase and the aqueous phase were mixed at a certain ratio to form a liposome;
(4) the liposome obtained in step "3" was subjected to granule sizing to afford a liposome with a relatively uniform grain size;
(5) the liposome obtained in step "4" was dialyzed, and the trehalose solution obtained in step "2" was used as the dialysate; and
(6) the liposome solution obtained in step "5" was freeze-dried or spray-dried to afford solid powder.

When used, the solid powder was re-dissolved to afford a paclitaxel cationic liposome injection, so that the osmotic pressure of the resulting injection was equal to the plasma osmotic pressure. The "paclitaxel cationic liposome injections" used in the subsequent examples were all obtained by re-dissolving the products of Example 1. Prescriptions 1-1, 1-2, and 1-3 had substantially the same physical and chemical properties and pharmacological and clinical effects.

The paclitaxel cationic liposome injections used in Examples 2 to 4 were obtained by re-dissolving the product 1-2 of Example 1, and were simplified as "A1". The paclitaxel cationic liposome injections used in Examples 5 and 6 were obtained by re-dissolving the product 1-1 of Example 1, and were simplified as "A2".

**Example 2: Inhibitory Effect of Combination of Paclitaxel Cationic Liposome for Injection and Cisplatin (DDP) on Prostate Cancer RM-1 Allografts in Mice**

Experimental animal: female C57/BL6 mice provided by Beijing Vital River Laboratory Animal Technology Co., Ltd.

Experimental method: Female C57/BL6 mice were subcutaneously inoculated with mouse prostate RM-1 cells. Six days after inoculation, the mice were grouped when the mean tumor volume was about 140 mm$^3$ (d0). Drugs were administered intravenously (except that DDP was administered intraperitoneally) at d0, d2, d4, d6, d8, and d10 for a total of 6 times. The experiment was terminated at d15. In the A1 high-dose group, A1 was administered at a dose of 12 mg/kg for the first three times and 6 mg/kg for the last three times, with a cumulative dose of 54 mg/kg; in the A1 low-dose group, A1 was administered at a dose of 6 mg/kg for six times, with a cumulative dose of 36 mg/kg; in the DPP group, DPP was administered at a dose of 1 mg/kg for six times, with a cumulative dose of 6 mg/kg; in the vehicle control group, 5% glucose injection (at an equal volume to A1 high-dose group) was administered; in the A1+DDP-1 group, A1 was administered at a dose of 6 mg/kg for six times, with a cumulative dose of 36 mg/kg, and DPP was administered at a dose of 1 mg/kg for six times, with a cumulative dose of 6 mg/kg; in the A1+DDP-2 group, A1 was administered at a dose of 12 mg/kg for the first three times and 6 mg/kg for the last three times with a cumulative dose of 54 mg/kg, and DPP was administered at a dose of 1 mg/kg for six times, with a cumulative dose of 6 mg/kg. During the experiment, the body weight and tumor volume of animals were monitored three times a week, and the values of the tumor volume (TV), relative tumor volume (RTV), and relative tumor proliferation rate (T/C) were calculated.

The related calculation formulae were as follows:

Tumor volume (TV) = $1/2 \times a \times b^2$, where a and b represented the major and minor diameters of the tumor, respectively.

RTV = TVt / TV$_0$. TV$_0$ was the tumor volume measured when grouping for administration (*i.e.* d0) and TVt was the tumor volume at each measurement.

T/C(%) = (TRTV / CRTV) $\times$ 100% (TRTV: RTV in the treatment group; CRTV: RTV in the vehicle control group).

Methods for drug preparation: A1: the paclitaxel cationic liposome was dissolved by adding water for injection, and then diluted with glucose and sodium chloride injection to the desired concentration; DDP: the DDP powder was dissolved in 5% glucose injection and subjected to ultrasound to formulate the desired concentration.

Experimental results: Compared with the vehicle control group (Vehicle), the prostate cancer RM-1 tumor growth in mice could be significantly inhibited in each of the treatment groups (P<0.05). Compared with the single DDP group, the A1 high- or low-dose + DDP group exhibited a significantly enhanced inhibitory effect on tumors (*P*<0.01). The specific results were listed in Table 1.

**Table 1: Inhibitory Effect of Combination with DDP on Prostate Cancer RM-1 Allografts in Mice ( $\bar{x} \pm$ sd, n=9)**

| Group | Cumulative Dose (mg/kg) | Dosing | Tumor Volume (mm$^3$) d0 | Tumor Volume (mm$^3$) d15 | RTV | T/C (%) |
|---|---|---|---|---|---|---|
| Vehicle | - | | 145.2±23.4 | 4684.7±1333.4 | 32.3±8.1 | - |
| A1 | 36 | Dosing at d0, d2, d4, d6, d8, d10 DDP (i.p) Others (i.v) | 141.8±21.1 | 2704.7±678.1** | 19.5±6.0** | 60.5 |
| A1 | 54 | | 136.7±20.1 | 2169.2±634.2** | 15.9±4.0** | 49.2 |
| A1+DDP-1 | 36/6 | | 144.2±22.2 | 2151.5±416.4**★★# | 12.1±8.1*★# | 37.5 |
| A1+DDP-2 | 54/6 | | 144.3±22.3 | 1471.8±578.4**★★ | 9.0±4.5*★★## # | 28.0 |
| DDP 1 mg/kg | 6 | | 145.1±22.8 | 3494.6±710.9* | 24.9±7.6 | 77.1 |

* p <0.05, ** p <0.01 compared to the Vehicle control group;
#p<0.05, ##p<0.01 compared to A1 at the same dose;
*p<0.05, **p<0.01 compared to DDP at the same dose

**Example 3: Inhibitory Effect of Paclitaxel Cationic Liposome for Injection on Human Pancreatic Cancer CFPAC-1 Xenografts in Mice**

Experimental animal: female Nu/Nu nude mice provided by Beijing Vital River Laboratory Animal Technology Co., Ltd.

Experimental method: Female Nu/Nu nude mice were subcutaneously inoculated with human pancreatic cancer CFPAC-1 cells. 5 days after inoculation, the mice were divided into 7 groups when the mean tumor volume was 228 mm$^3$ (d0), namely, the vehicle control group (5% glucose injection), the 6 mg/kg of A1 group, the 3 mg/kg or 6 mg/kg of A1 + 20 mg/kg of gemcitabine for injection(GEM) group, the 6 mg/kg of paclitaxel injection (Taxol) group, the 6 mg/kg of Taxol + 20 mg/kg of GEM group, and the 20 mg/kg of GEM group, respectively. There were 10 mice in each group. Drugs were administered intravenously (except that the GEM was administered intraperitoneally for 5 times (at the first five time points)) at d0, d3, d6, d9, d13, and d16 for a total of 6 times. After the last administration, the mice were further observed for 11 *d (i.e.* d27) and the experiment was terminated. During the experiment, the body weight and tumor volume of animals were monitored twice a week, and the tumor volume (TV) was calculated. At the end of the experiment, the tumor weight was weighed and the tumor weight inhibition rate was calculated.

The related calculation formulae were as follows:

Tumor volume (TV) = $1/2 \times a \times b^2$, where a and b represented the major and minor diameters of the tumor, respectively.

Tumor weight inhibition rate (%) = (1 - tumor weight in treatment group/ tumor weight in vehicle control group) $\times$ 100%.

Methods for drug preparation: A1 was prepared using the same method as in Example 2. The gemcitabine for injection was dissolved in saline to achieve the desired concentration. Paclitaxel injection (Taxol) was diluted with saline to the desired concentration.

Experimental results: Compared with the vehicle control group, administration of 6 mg/kg of A1 alone could significantly inhibit the human pancreatic cancer CFPAC-1 tumor growth (*P*<0.01), and a combination of A1 and gemcitabine exhibited a stronger inhibitory effect on the tumor than administration of A1 or gemcitabine alone (P<0.01). Administration of 6 mg/kg of A1 either alone or in combination with gemcitabine exhibited a significantly enhanced inhibitory effect on the tumor (P<0.01), as compared to the paclitaxel injection (Taxol) under the same administration conditions. The specific results were listed in Table 2.

**Table 2: Inhibitory Effect on Human Pancreatic Cancer CFPAC-1 Xenografts ( $\overline{x} \pm$ sd, n=10)**

| Group | Dose (mg/kg) | Dosing | Tumor Volume (mm³) | | Tumor Weight (g) | Tumor Weight Inhibition Rate (%) |
| --- | --- | --- | --- | --- | --- | --- |
| | | | d0 | d27 | | |
| Vehicle | - | Dosing at d0, d3, d6, d9, d13, d16 GEM (ip) Others (iv) | 226.3±50.1 | 1599.4±607.7 | 1.5968±0.6144 | - |
| A1 | 6 | | 227.6±65.9 | 412.8±237.0**▲▲△△ | 0.4176±0.3314**▲▲△ | 73.8 |
| A1+GME | 3/20 | | 230.2±58.6 | 52.5±42.2**★★ | 0.0221±0.0138**★★ | 98.6 |
| A1+GME | 6/20 | | 227.0±52.0 | 26.3:1:24.6**△△★★## | 0.0104±0.0127**△△★★## | 99.4 |
| Taxol | 6 | | 227.0±52.0 | 854.3±266.4** | 0.6905±0.1243** | 56.8 |
| Taxol+GE M | 6/20 | | 227.0±52.0 | 404.3±190.6**★ | 0.3688±0.1937**★★ | 76.9 |
| GME | 20 | | 227.3±52.8 | 720.9±152.8** | 0.8238±0.3253** | 48.4 |

*p<0.05, **p<0.01 compared to the vehicle control group;
△p<0.05 compared to Taxol at the same dose; *p<0.05, **p<0.01 compared to GEM;
##p<0.01, A1 + GEM compared to A1 at the same dose

### Example 4: Inhibitory Effect of Paclitaxel Cationic Liposome for Injection on Human Triple-Negative Breast Cancer MDA-MB-231 Xenografts in Mice

Experimental animal: female NOD/SCID mice provided by Beijing Vital River Laboratory Animal Technology Co., Ltd.

Experimental method: Female NOD/SCID mice were subcutaneously inoculated with human triple-negative breast cancer MDA-MB-231 cells. 7 days after inoculation, the mice were divided into 7 groups when the mean tumor volume was 120 to 130 mm³ (d0), namely, the vehicle control group (5% glucose injection), the 6 mg/kg of A1 group, the 3 mg/kg or 6 mg/kg of A1 + 400 mg/kg of capecitabine for injection(CAP) group, the 6 mg/kg of paclitaxel injection (Taxol) group, the 6 mg/kg of Taxol + 400 mg/kg of CAP group, and the 400 mg/kg of CAP group, respectively. The CAP for injection was administered intragastrically twice a week and the other drugs were administered intravenously twice a week for a total of 7 times (d0, d3, d7, d10, d14, d17, and d21). The experiment was terminated at d28. During the experiment, the body weight and tumor volume of animals were monitored twice a week, and the tumor volume (TV) was calculated. At the end of the experiment, the tumor weight was weighed and the tumor weight inhibition rate was calculated.

The related calculation formulae were as follows:

Tumor volume (TV) = $1/2 \times a \times b^2$, where a and b represented the major and minor diameters of the tumor, respectively.

Tumor weight inhibition rate (%) = (1 - tumor weight in treatment group / tumor weight in vehicle control group) $\times$ 100%.

Methods for drug preparation: CAP was prepared by diluting with water for injection to the desired concentration, and the rest of drugs were prepared according to the method described in Example 3.

Experimental results: Compared with the vehicle control group, administration of 6 mg/kg of A1 alone could significantly inhibit the human triple-negative breast cancer MDA-MB-231 tumor growth *(P<0.05),* and a combination of A1 and CAP exhibited a stronger inhibitory effect on the tumor than administration of A1 or CAP alone (P<0.05). Administration of 6 mg/kg of A1 either alone or in combination with CAP exhibited a significantly enhanced inhibitory effect on the tumor (*P*<0.05), as compared to the Taxol under the same administration conditions. The specific results were listed in Table 3.

Table 3: Inhibitory Effect on Human Triple-Negative Breast Cancer MDA-MB-231 Xenografts ( $\overline{x} \pm$ sd, n=10)

| Group | Dose (mg/kg) | Dosing | Tumor Volume (mm$^3$) | | Tumor Weight (g) | Tumor Weight Inhibition Rate (%) |
|---|---|---|---|---|---|---|
| | | | d0 | d20 | | |
| Vehicle | - | Dosing at d0, d3, d7, d10, d14, d17, d21, CAP (po) Others (iv) | 129.1±11.6 | 2599.7±531.6 | 2.0547±0.1379 | - |
| A1 | 6 | | 124.4±18.9 | 1326.2±520.3**▲ | 1.0908±0.2914**▲▲△ | 46.9 |
| A1+CAP | 3/400 | | 124.1±17.9 | 1375.7±341.3** | 1.1727±0.2492**★★ | 42.9 |
| A1+CAP | 6/400 | | 123.8±17.9 | 748.6±400.0**★★△# | 0.6565±0.1935**★★△## | 68.0 |
| Taxol | 6 | | 123.9±18.1 | 1659.9±607.9** | 1.4165±0.2541** | 31.1 |
| Taxol+CAP | 6/400 | | 124.1±19.3 | 1128.4±210.0**★ | 0.9434±0.1890**★★ | 54.1 |
| CAP | 400 | | 124.5±19.4 | 1708.3±564.8** | 1.3679±0.2649** | 33.4 |

*p<0.05, **p<0.01 compared to the vehicle control group;
△p<0.05, △△p<0.01 compared to Taxol; *p<0.05, **p<0.01 compared to CAP;
#p<0.05, ##p<0.01, A1+ CAP compared to A1 at the same dose

**Example 5: Clinical Study on First-Line Therapy of Gastric Cancer, Pancreatic Cancer, and Colorectal Cancer with Liver Metastases by Arterial Infusion of Paclitaxel Cationic Liposome Injection**

This study was a single-arm, open-label and single-center investigator-initiated clinical trial (IIT), into which patients with treatment-naive liver metastases (including, but not limited to, gastric cancer with liver metastases, colorectal cancer with liver metastases, and pancreatic cancer with liver metastases), whose primary cancers were histopathologically or cytologically confirmed, were enrolled and administered with different doses of paclitaxel cationic liposome injection via arterial infusion plus fixed doses of XELOX regimen. This study was intended to explore the safety and tolerability of the combined regimen, determine the optimal dose of the paclitaxel cationic liposome injection in the combined regimen, while evaluating the efficacy and observing the pharmacokinetic characteristics.

**I. Study Design**

This study was divided into a dose-escalation phase and a dose-expansion phase.

**1. Dose-escalation phase**

(1) Design of trial

The study included a screening period, a treatment period and an end-of-study follow-up period.

After signing the Informed Consent Form and completing all baseline assessments within the screening period, subjects who were eligible would be enrolled into the treatment period, and treated by administering the paclitaxel cationic liposome injection in combination with the XELOX regimen. The doses of the paclitaxel cationic liposome injection would be gradually escalated from a low-dose level to a high-dose level. The dose administered to the subjects was the current or intended dose at the entry into this study. The dosing regimen followed once every three weeks. The first cycle in the treatment period was the DLT (Dose-Limiting Toxicity) observation period. Subjects who had completed the 3-week treatment and observation could proceed to the next cycle of administration until tumor relapse/progression or death occurred, the subject or his/her legal representative requested to withdraw from the study, an alternative treatment means was applied, or the entire study was completed (whichever occurred first). The administration was expected to last for 6 to 8 cycles. The efficacy was evaluated once every 2 cycles (6 weeks) during treatment. All subjects were subjected to the collection of blood samples for PK (pharmacokinetics) analysis at different time points before and after the administration according to the protocol, and completed the relevant examinations specified in the protocol during the study, so as to observe the safety and tolerability. The end-of-treatment follow-up visit started at day 30 after the last dose.

(2) Dose escalation protocol

Paclitaxel cationic liposome: 11 mg/m$^2$ was taken as the initial dose (on a paclitaxel basis), and a total of five dose groups (11 mg/m$^2$, 22 mg/m$^2$, 33 mg/m$^2$, 44 mg/m$^2$, and 55 mg/m$^2$) were designed.

The corresponding treatment of subjects who experienced DLT would be determined by the investigators according to the standard for clinical diagnosis and treatment, and these subjects might proceed to participate in the next cycle of administration by either maintaining at the original dose or down-regulating it by one dose level. The dose was not allowed to increase for the same subject.

XELOX regimen: oxaliplatin administered at a dose of 130 mg/m$^2$ via intravenous infusion at D1; and capecitabine orally administered at a dose of 1000 mg/m$^2$ at D1 to D14.

(3) Extended dosing after completion of DLT observation period

The corresponding treatment of subjects who experienced DLT would be determined by the investigators according to the standard for clinical diagnosis and treatment, and these subjects might proceed to the next cycle of administration by delaying the administration for no more than 2 weeks, maintaining at the original dose or down-regulating it by one dose level. The dose was not allowed to up-regulate for the same subject.

Once the MTD (maximum tolerated dose) of paclitaxel cationic liposome injection is identified, MTD can be selected as RP2D (recommended phase II dose). If the MTD of paclitaxel cationic liposome injection was not identified, RP2D would be determined by the investigators after internal discussion based on the research results in the DLT observation period in the dose-escalation phase. Furthermore, RP2D was selected for expansion in the dose-expansion phase.

**2. Dose-expansion phase**

Dose expansion might be conducted, if necessary, based on the data such as safety, tolerability, and efficacy obtained from the dose-escalation phase.

One to three types of cancer were selected for dose expansion, including but not limited to gastric cancer with liver metastases, colorectal cancer with liver metastases, pancreatic cancer with liver metastases, etc. The specific cohorts to be expanded could be determined by the investigators through internal discussion after the expansion dose was determined, and 5 to 15 cases were expanded for each cohort.

**II. Study Population**

**(I) Inclusion criteria**

Subjects who met all of the following criteria were eligible for the enrollment of this study:

1. Subjects aged from 18 to 75 (inclusive), regardless of gender.
2. Subjects with treatment-naive liver metastases, including but not limited to, gastric cancer with liver metastases, colorectal cancer with liver metastases, and pancreatic cancer with liver metastases, primary cancers were histopathologically or cytologically confirmed.

3. If the liver metastasis was a single measurable target lesion, the diameter of the single target lesion was required to be ≥2 cm. If the liver metastasis was two or more measurable target lesions, at least two target lesions were required to have a diameter of ≥1 cm.

4. ECOG performance status score: 0 to 2.

5. Life expectancy of over 3 months.

### III. Efficacy Evaluation Indicators

The efficacy was evaluated by CT/MRI according to the RECIST1.1/mRECIST standard.

1. Main efficacy endpoints:

Overall response rate (ORR): It was defined as the proportion of subjects in the study whose best overall response were CR or PR as evaluated by the investigators according to RECIST v1.1/mRECIST.

Disease control rate (DCR): It was defined as the proportion of subjects with responses of CR or PR, and stable disease (SD) (*i.e.,* CR+PR+SD) at the time point of best overall response during the period from the time of first administration of the study drug to the end of study, as evaluated according to the RECIST 1.1/mRECIST.

2. Exploratory efficacy endpoint:

Change rate of the target lesions in the arterial infusion area: All measurable lesions (it was required that if there was only one target lesion in the arterial infusion area, the target lesion had a diameter of ≥2 cm; and if there were two or more target lesions, at least two target lesions had a diameter of ≥1 cm) in the arterial infusion area were measured according to RECIST v1.1/mRECIST. The sum of the diameters of the target lesions in the blood supply area was calculated as the baseline data, and the change rate of the sum of the diameters of the target lesions in the blood supply area at each time point of efficacy evaluation was calculated. When the lesions in the blood supply area were shrunk and then regrew, the change rate relative to the sum of the minimum diameters of the target lesions in the blood supply area was calculated.

3. Safety endpoints

AE (Adverse Event) and SAE (Serious Adverse Event/Reaction) were used as the evaluation indicators of safety.

### IV. Research Results

Arterial infusion of the paclitaxel cationic liposome injection in combination with the systemic therapy (e.g. XELOX regimen) exhibited good effects on treatment of liver metastases of advanced solid tumors (for example, gastric cancer, pancreatic cancer, and colorectal cancer), could improve the efficacy and reduce the incidence of adverse reactions, and had a good prospect for clinical application.

In this Example, a total of 5 patients (the first 5 cases in Table 4) were enrolled, including one case of gastric cancer with liver metastases and 4 cases of colorectal cancer with liver metastases, and none of them had received any prior systemic therapy for metastatic diseases. One case of colorectal liver metastases was undergoing screening evaluations (the last case in Table 4 with the subject number pending). The detailed information was listed in Table 4.

Table 4

| Dose Group | Subject No. | Gender and Age | Diagnosis | Tumor Staging (First Diagnosis) | Previous Surgery | Previous Antitumor Therapy |
|---|---|---|---|---|---|---|
| 11 mg/m² | 01002 | Male 63 years old | Gastric cancer with liver metastases | Stage IIIA (T4aN2M0) | Laparoscopic radical resection of gastric cancer (2022-5-6) | 4-Cycles of SOX regimen on 2022-7-4, 7-25, 8-16, and 9-1 (Tegafur 60 mg BID D1-14 + oxaliplatin 150 mg D1) |

(continued)

| Dose Group | Subject No. | Gender and Age | Diagnosis | Tumor Staging (First Diagnosis) | Previous Surgery | Previous Antitumor Therapy |
|---|---|---|---|---|---|---|
| 22 mg/m$^2$ | 01003 | Male 58 years old | Sigmoid colon cancer with liver metastases | Stage IIIA (pT2N1M0) | Laparoscopic radical resection of sigmoid colon cancer (2019-6) | 1 Cycle of XELOX regimen after surgery in 2019.6 (capecitabine + oxaliplatin) |
| 22 mg/m$^2$ | 01004 | Male 61 years old | Ascending colon cancer with liver metastases | Stage IV (pT3N0M1a) | Laparoscopic right hemicolectomy of colon cancer (2023-5) | None |
| 22 mg/m$^2$ | 01005 | Male 57 years old | Rectal cancer with liver, lung, and bone metastases | Stage IV (TxNxM1) | None | None |
| 33 mg/m$^2$ | 01006 | Male 59 years old | Sigmoid colon cancer with liver metastases | Stage IV (TxNxM1) | None | None |
| 33 mg/m$^2$ | TBD | Male 37 years old | Colon cancer with liver metastases | Stage IV (TxNxM1) | None | None |

Table 5

| Dose Group | Subject No. | Diagnosis | Target Lesion | Non-Target Lesion | First Infusion Time of study drug | Tumor Response Evaluation (1st time) | Tumor Response Evaluation (2nd time) | Tumor Response Evaluation (3rd time) |
|---|---|---|---|---|---|---|---|---|
| 11 mg/m$^2$ | 01002 | Gastric cancer liver with metastases | Hepatic segment VI (1): 11.0 mm Hepatic segment VIII (2): 18.3 mm | None | 2023/4/20 | **SD (-1%)** Target 1: 10.94 mm Target 2: 18.1 mm | **SD (-21%)** Target 1: 8.19 mm Target 2: 15.01 mm | **PD (+50%)** Target 1: 12.79 mm Target 2: 22.09 mm |
| 22 mg/m$^2$ | 01003 | Sigmoid colon cancer with liver metastases | Hepatic interlobar junctional zone (1): 91.37 mm Inferior portion of right hepatic lobe (2): | None | 2023/6/26 | **SD (-15%)** Target 1: 86.39 mm Target 2: 0 | **SD (-15%)** Target 1: 86.39 mm Target 2: 0 | **PR (-72%)** Target 1: 28.5 mm Target 2: 0 |

(continued)

| Dose Group | Subject No. | Diagnosis | Target Lesion | Non-Target Lesion | First Infusion Time of study drug | Tumor Response Evaluation (1st time) | Tumor Response Evaluation (2nd time) | Tumor Response Evaluation (3rd time) |
|---|---|---|---|---|---|---|---|---|
| 22 mg/m² | 01004 | Colon cancer with liver metastases | Posterior lobe of liver (1): 13.83 mm Right lobe of liver (2): 13.17 mm | Right lobe of liver | 2023/6/29 | **PR (-51%)** Target 1: 5.94 mm Target 2: 5.94 mm | **PR (-59.8%)** Target 1: 5.36 mm Target 2: 4.46 mm | **PR (-89%)** Target 1: 3.74 mm Target 2: 0 |
| 22 mg/m² | 01005 | Rectal cancer with liver metastases | Rectosigmoid junction (1): 12.46 mm Hepatic segment IV (2): 43.22 mm Hepatic segment IV (3): 11.16 mm RUL posterior s:egment (4): 13.97 mm | Centrum L3, L4 | 2023/8/22 | **SD (-10%)** Target 1: 9.92 mm Target 2: 38.83 mm Target 3: 11.79 mm Target 4: 13 mm Non-target: present | SD (-16%) Target 1: 8.01 mm Target 2: 35.94 mm Target 3: 11.76 mm Target 4: 12.47 mm Non-target: present | |
| 33 mg/m² | 01006 | Colon cancer with liver metastases | Right lobe of liver: 26.7 mm Right lobe of liver: 16.3 mm | Right lobe of liver Descending-sigmoid junction | 2023/10/31 | **PR (-37%)** Target 1: 15.8 mm Target 2: 11.2 mm | | |

The results of best overall response were shown in Table 5. A total of 4 cases were evaluable for efficacy, of which 2 cases were evaluated as SD and 2 cases were evaluated as PR (both 01003 and 01004 subjects achieved CR evaluated by mRECIST), namely, ORR was 50% and DCR was 100%.

Safety and tolerability: No DLT was observed in 11 to 33 mg/m² dose groups. The safety and tolerability were favorable, and no abnormal safety signals were found. All the drug-related AEs currently reported were Grade 1 to 2 (FIG. 1, the incidence indicated by the vertical axis), and were recoverable after symptomatic treatment. Drug-related AEs (included possibly irrelevant) included the decreased platelet count, elevated procalcitonin, fever, nausea, increased white blood cell count, and decreased lymphocyte count.

Note: the adverse events in clinical studies were divided into the following five grades:

Grade 1: mild, asymptomatic or mild symptoms only observed in clinical or diagnostic practice; no need of treatment.
Grade 2: moderate, need of minimal, local or noninvasive treatment; confined age-appropriate instrumental activities of daily lives.
Grade 3: severe or medically significant but not immediately life-threatening, causing hospitalization or prolonged hospitalization, disabling, confined self-care ADL.
Grade 4: life-threatening, need of treatment.
Grade 5: AE-related death.

**Example 6: Clinical Study on Treatment of Advanced Solid Tumor by Arterial Infusion of Paclitaxel Cationic Liposome Injection**

This study was a single-arm, open-label and multicenter Phase 1 study, into which patients have histopathologically or cytologically confirmed advanced solid tumors were enrolled and administered with different doses of paclitaxel cationic liposome injection via arterial infusion. This study was intended to explore the safety and tolerability of the paclitaxel cationic liposome injection administered alone via arterial infusion, while evaluating the efficacy and observing the pharmacokinetic characteristics.

**I. Study Design**

This study was divided into a dose-escalation phase and a dose-expansion phase.

**1. Dose-escalation phase**

(1) Study Design

The dose-escalation phase included a screening period (within 28 days), a treatment period, and a PFS follow-up period. After signing the Informed Consent Form and completing all baseline assessments within the screening period, subjects who were eligible would be enrolled into the treatment period. The doses of the paclitaxel cationic liposome injection would be gradually escalated from a low-dose level to a high-dose level with every 3W (21 days) as a cycle. DLT was observed in the first dosing cycle. The subjects were subjected to the collection of blood samples for PK analysis at different time points before and after the administration according to the protocol, and completed the relevant examinations specified in the protocol during the treatment, so as to observe the safety, tolerability, and efficacy. The same subject could only receive one dose level administration and treatment schedule during treatment period. The dosing was expected to last for 6 to 8 cycles. The efficacy was evaluated once every 2 cycles (6 weeks) during treatment period.

(2) Dose escalation schedule

Paclitaxel cationic liposome injection: 24 mg/m$^2$ was taken as the initial dose (on a paclitaxel basis), and a total of five dose groups (24 mg/m$^2$, 36 mg/m$^2$, 48 mg/m$^2$, 60 mg/m$^2$, and 70 mg/m$^2$) were designed.

The corresponding treatment of subjects who experienced DLT would be determined by the investigators according to the standard for clinical diagnosis and treatment, and these subjects might proceed to the next cycle of administration by either maintaining at the original dose or down-regulating it by one dose level. The dose was not allowed to up-regulate for the same subject.

(3) Extended dosing after completion of DLT observation period

The corresponding treatment of subjects who experienced DLT would be determined by the investigators according to the standard for clinical diagnosis and treatment, and these subjects might proceed to the next cycle of administration by delaying the administration for no more than 2 weeks, maintaining at the original dose or down-regulating it by one dose level. The dose was not allowed to up-regulate for the same subject.

Once the maximum tolerated dose(MTD) of paclitaxel cationic liposome injection was identified, MTD can be selected as recommended phase II dose (RP2D). If the MTD of paclitaxel cationic liposome injection was not identified, RP2D would be determined by the investigators after internal discussion based on the results in the DLT observation period in the dose-escalation phase. Furthermore, RP2D was selected for expansion in the dose-expansion phase.

**2. Dose-expansion phase**

Dose expansion might be conducted, if necessary, based on the results such as safety, tolerability, and efficacy obtained from the dose-escalation phase. One to three types of tumor were selected for dose expansion, including but not limited to gastric cancer with liver metastases, colorectal cancer with liver metastases, pancreatic cancer with liver metastases, etc. The specific cohorts to be expanded could be determined by the investigators through internal discussion after the expansion dose was determined, and 5 to 15 cases were expanded for each cohort.

**II. Study Population**

**(I) Inclusion criteria**

Subjects who met all of the following criteria were eligible for the enrollment of this study:

1. Subjects aged from 18 to 75 (inclusive), regardless of gender.
2. Subjects have histopathologically or cytologically confirmed advanced solid tumor, which included, but was not limited to, the following types of tumor:

gastrointestinal tumors (including but not limited to: gastric cancer, liver cancer, cholangiocarcinoma, pancreatic cancer, colorectal cancer, etc.);
gynecological tumors (including but not limited to: ovarian cancer, endometrial cancer, etc.);
lung cancer; andsolid tumor with liver metastases.

3. Requirements for the target lesion: if there was only a single target lesion in the arterial infusion blood supply area, the diameter of the single target lesion was required to be ≥2 cm; if there were two or more target lesions in the blood supply area, at least two target lesions were required to have a diameter of ≥1 cm.
4. ECOG performance status: 0 to 2.
5. Life expectancy of over 3 months.

**III. Evaluation Endpoints**

The efficacy was evaluated by CT/MRI according to the RECIST 1.1 standard.
Cases planned to be enrolled in this study:

approximately 18 to 24 cases were enrolled in the dose-escalation phase;
the cases enrolled in the dose-expansion phase was decided by the investigators after internal discussion.

Efficacy evaluations:

Main efficacy endpoints:

Overall response rate (ORR): It was defined as the proportion of subjects in the study whose best overall responses were CR or PR as assessed by the investigators according to RECIST v1.1.
Disease control rate (DCR): It was defined as the proportion of subjects with responses of CR or PR, and stable disease (SD) *(i.e.,* CR+PR+SD) at the time point of best overall response during the period from the time of first administration of the study drug to the end of study, as evaluated according to the RECIST 1.1 standard.
Progression-free survival (PFS): It was defined as the period from the time of first administration of the study drug to the date of first documented disease progression (PD) or the date of death, whichever occurred first.
Duration of response (DoR): It was defined as the period from the time when the tumor was firstly assessed as CR or PR to the time when the tumor was firstly assessed as PD or the subject died from any cause.

Safety endpoints

Adverse Event (AE) and Serious Adverse Event/Reaction (SAE) were used as the evaluation endpoints of safety.

IV. Results

Arterial infusion of paclitaxel cationic liposome injection exhibited favorable efficacy on treatment of advanced solid tumors (for example, gastric cancer, pancreatic cancer, and colorectal cancer with liver metastases), could improve the efficacy and reduce the incidence of adverse reactions, and had a good prospect for clinical application.

**Example 7: Clinical Study on Evaluations of Safety, Tolerability and Pharmacokinetic Characteristics of Paclitaxel Cationic Liposome Injection for Treatment of Patients with Advanced Solid Tumors via Transcatheter Arterial Infusion**

**I. Primary and Secondary Objectives and Endpoints**

### 1. Primary objective

(1) Evaluating safety and tolerability of paclitaxel cationic liposome injection for treatment of advanced solid tumors via transcatheter arterial infusion.
(2) Determining the maximum tolerated dose (MTD) and/or recommended dose for expansion (RDE) of the paclitaxel cationic liposome injection for treatment of advanced solid tumors via transcatheter arterial infusion.

### Primary endpoints

(1) Frequency and severity of adverse events (AEs) and serious adverse events (SAEs) (according to NCI CTCAE 5.0).
(2) Frequency of dose-limiting toxicity (DLT).

### 2. Secondary objective

(1) Evaluating pharmacokinetic (PK) characteristics of paclitaxel cationic liposome injection for treatment of advanced solid tumors via transcatheter arterial infusion.
(2) Evaluating the preliminary antitumor activity of paclitaxel cationic liposome injection for treatment of advanced solid tumors via transcatheter arterial infusion.

### Secondary endpoints

(1) Pharmacokinetic indicators: PK parameters including but not limited to $AUC_{0-t}$, $AUC_{0-\infty}$, $C_{max}$, $K_{el}$, $T_{max}$, Vd, $t_{1/2}$, CL, etc.
(2) Efficacy endpoints: overall response rate (ORR), disease control rate (DCR), duration of response (DOR), progression-free survival (PFS), and overall survival (OS).

### II. Overall Study Design

This study included two phases: Phase Ia (dose-escalation and expansion phases) and Phase Ib (cohort-expansion phase).

The dose-escalation phase included a screening period (0 to approx. 28 days), a treatment period (a DLT observation period and a subsequent treatment period) and a follow-up period (safety follow-up visit and survival follow-up visit). The first cycle (21 days), in which the subjects received the study drug, was the DLT observation period. After the DLT observation period, the subjects would proceed to the subsequent treatment period, provided that they did not experience DLT. The dose-expansion phase and the cohort-expansion phase included a screening period (0 to approx. 28 days), a treatment period, and a follow-up period (safety follow-up visit and survival follow-up visit). The paclitaxel cationic liposome injection was administered via arterial infusion at day 1 of each cycle, with three weeks as a treatment cycle. Eligible subjects would experience 4- to 6-cycle study treatment. Treatment might be early terminated if any of the following cases occurred: disease progression, intolerable toxicity, initiation of a new antitumor therapy, withdrawal of Informed Consent Form, loss of follow-up, or death, or other criteria for discontinuation of treatment (whichever occurred first). If a subject experienced the first disease progression but the investigators assessed that the subject could still benefit from continuous treatment and the subject was willing to continue, the subject might proceed with 1-to 2-cycle study treatment until the disease progression occurred again.

During the study, the subjects would undergo efficacy evaluations once every 6 weeks $\pm$ 7 days since the first dose. For subjects who discontinued treatment for other reasons than disease progression, the efficacy evaluations should be continued until the following cases occurred: disease progression or initiation of a new antitumor therapy, participant's withdrawal of Informed Consent Form, loss of follow-up, death, etc. (whichever occurred first).

**1. Dose-escalation phase**

The initial dose of the paclitaxel cationic liposome injection was set at 22 mg/m$^2$, and might be appropriately adjusted after discussion by the SMC (Scientific Monitoring Committee established for this trial) based on the data obtained from the previous studies on the treatment with this product via arterial infusion. The maximum dose level was currently estimated at 66 mg/m$^2$. If this dose was safe and tolerable, the investigators and the sponsor might discuss and decide whether or not to escalate to a higher dose level. If the subject s in the low-dose level group did not experience DLT in the DLT observation period and this dose level demonstrated good safety and tolerability, these subjects might receive a higher dose of the study drug subsequently after the SMC's discussion.

The specific dose groups in the dose-escalation phase were designed according to the modified Fibonacci approach and shown in the table below.

Table 6 Design of Dose Groups in Dose-Escalation Phase (dosing once every 3 weeks)

| Dose group | 1 | 2 | 3 | 4 | 5 |
|---|---|---|---|---|---|
| Escalation rate | - | 50% | 33% | 25% | 20% |
| Dose (mg/m$^2$) | 22 | 33 | 44 | 55 | 66 |
| Mode of administration | Chemotherapy via transcatheter arterial infusion | | | | |

Apart from the initial dose, the escalated doses could be discussed by the investigators and the sponsor based on the obtained data such as drug safety and PK to decide whether or not to proceed to the next dose level and whether or not to adjust the dosing levels or dosing regimens for the other dose groups.

In this study, the first dose group followed the accelerated dose titration design, and the subsequent dose groups followed the "3+3" dose-escalation design. DLT of the paclitaxel cationic liposome injection was observed in the first cycle (within 21 days). If the subject s in the accelerated titration group experienced moderate toxicity (including drug-related AEs of the following levels that needed to be intervened: a single occurrence of other AEs of Grade 3 that were not DLT, or two occurrences of AEs of Grade 2 or higher), not only this dose group but also the subsequent dose groups followed the "3+3" design.

**"3+3" Dose-escalation design:**

Three cases were enrolled in each dose group. After DLT observation, the cases were evaluated to determine whether the next dose group could be proceed based on the following rules:

(1) three subjects were enrolled in the first dose group (or a particular dose group) and completed the DLT observation;
(2) if none of the three subjects in a dose group experienced DLT, the dose would be escalated to the next dose group;
(3) if two or more of the three subjects in a dose group experienced DLT, the dose would be deescalated to the level of the previous dose group;
(4) if one of the three subjects in a dose group experienced DLT, three more subjects would be enrolled in this dose group; if 1/6 subjects experienced DLT, the dose would be escalated to the next dose group; if 2/6 or more subject s experienced DLT, the dose would be deescalated to the previous dose group;
(5) if the dose was deescalated to the previous dose group and there were only 3 subjects in this dose group, three more subjects would be enrolled. If there had been 6 subjects in this dose group, the dose-escalation was terminated and this dose was MTD.

**Maximum tolerated dose (MTD):**

MTD: It was defined as the maximum dose at which 33% or less of subjects experienced DLT. In general, if two or more of 3 subjects enrolled at a dose level experienced DLT, the dose immediately preceding this dose was MTD; if one of 3 subject s enrolled at a dose level experienced DLT and if 2/6 or more of the subject s experienced DLT after enrollment of three more subjects at the same dose level, the dose immediately preceding this dose was MTD.

A new dose might be selected for trial between the preceding dose and "the intolerable dose", if necessary.

**2. Dose-expansion phase**

Based on the data such as PK, safety, tolerability, and efficacy obtained from the dose-escalation study, 1 to 3 recommended dose groups might be selected and the enrollment could be expanded to 15 to 30 subjects (including those in the corresponding dose group(s) in the dose-escalation phase).

### 3. Cohort-expansion phase

After the Recommended Dose for Expansion (RDE) was determined, 1 to 3 types of tumors that may benefit were selected for cohort expansion, including but not limited to: liver cancer, liver metastases, pancreatic cancer, lung cancer, etc. The specific population with the indications and the sample size could be determined by the SMC after full discussion.

### III. Dose-Limiting Toxicity (DLT)

The dose-limiting toxicity was defined as an adverse event that occurred during the first cycle (21 days) in the dose-escalation phase, (definitely, probably or possibly) related to the paclitaxel cationic liposome injection, and met the following severity levels (in accordance with NCI CTCAE 5.0). Except for the concomitant therapies prohibited in the protocol, clinical diagnosis and treatment routines should be followed in the study, and adequate and appropriate symptomatic support should be provided in a timely manner.

| Toxicity | Determined as DLT in case of any of the following events: |
|---|---|
| Hematologic toxicity | ● Grade 4 neutropenia (absolute neutrophil count [ANC] <500/mm$^3$) persisting for more than 3 days<br>● ≥ Grade 3 febrile neutropenia (ANC<1000/mm$^3$ with a single axillary temperature of >38.3°C or a sustained axillary temperature of ≥38.0° C for over 1 h)<br>● Grade 4 thrombocytopenia (platelet count <25,000/mm$^3$) persisting for more than 5 days<br>● Grade 3 thrombocytopenia (platelet count <50,000/mm$^3$) with clinically significant bleeding<br>● Grade 4 anemia (life-threatening) |
| Non-hematologic toxicity | ● Toxicity at Grade 4 or higher<br>● Special monitoring: Grade 3 or higher thromboembolic events and arterial thromboembolism, Grade 1-2 cerebrovascular ischemia<br>● Other Grade 3 toxicities of non-special concern, except for:<br>  ○ Grade 3 fatigue, headache, nausea, diarrhea, vomiting, electrolyte imbalance (including hypokalemia, hypophosphatemia, hypocalcemia, etc.), which recovered to Grade 1 or baseline within 48 h after symptomatic treatment;<br>  O Grade 3 fever (>40°C, no more than 24 h), resolvable by symptomatic treatment<br>  O Non-clinical disease-related or isolated laboratory abnormalities determined by the investigators not to be clinical significant |
| Others | ● Other toxicities determined by the investigators to warrant permanent discontinuation of the study drug<br>● Late toxicities that occurred after the DLT observation period but might increase the risk to subjects |

If the following events occurred within the DLT observation period, the DLT observation period was deemed not to be completed and cases should be replaced:

- the dose administration was lower than 75% of the planned total dose due to non-DLT reasons;
- termination of drug administration due to non-DLT reasons.

### IV. Study Population

### Groups and subjects

Dose-escalation phase: 15 to 30 cases;
Dose-expansion phase: 1 to 3 recommended dose groups were selected and the enrollment were expanded to 15 to 30 subjects (including those in the corresponding dose group(s) in the dose-escalation phase);

Cohort expansion phase: to be determined (by the SMC after discussion based on the data obtained in the dose escalation and expansion phases).

**Inclusion criteria:**

1. age ≥18 years old;

2. histologically or cytologically diagnosed advanced solid tumors suitable for arterial infusion chemotherapy, including but not limited to the following types of tumor:

- gastrointestinal tumors (including but not limited to gastric cancer, liver cancer, cholangiocarcinoma, pancreatic cancer, colorectal cancer, etc.);
- gynecological tumors (including but not limited to ovarian cancer, endometrial cancer, etc.);
- non-small cell lung cancer;
- solid tumor with liver metastases;

3. at least one measurable lesion in the arterial infusion area according to RECIST 1.1;

4. if the most life-threatening lesions were in the arterial infusion area, limited lesions outside the arterial infusion area was acceptable at baseline;

5. Eastern Cooperative Oncology Group (ECOG) performance status score of 0 to 2;

6. life expectancy of at least 3 months;

7. adequate organ functions, with laboratory test results meeting the following criteria (without transfusion or hematopoietic growth factor therapy within 14 days):

a. absolute neutrophil count (ANC)≥1.5 × $10^9$/L

b. platelet count (PLT) ≥100 × $10^9$/L

c. hemoglobin (Hb) ≥90 g/L

d. total bilirubin (TBIL) ≤ 1.5 × upper limit of normal (ULN); TBIL ≤2 × ULN for subjects with liver metastasis or liver cancer

e. alanine aminotransferase (ALT), aspartate aminotransferase (AST) ≤2.5 × ULN; ALT, AST ≤5 × ULN for subjects with liver metastasis or liver cancer

f. creatinine clearance (Ccr)>50 mL/min (calculated according to the Cockcroft-Gault formula)

g. activated partial thromboplastin time (APTT) ≤1.5 × ULN, international normalized ratio (INR) ≤ 1.5 × ULN

8. fertile eligible subjects (male and female) had to agree to use reliable contraceptive methods (hormonal contraceptives, barrier methods, or abstinence) with their partners during the study and for at least 6 months after the last dose; female subjects of childbearing age had to have a negative blood pregnancy test within 7 days prior to enrollment;

9. having a full understanding of this clinical study and voluntarily signing a written Informed Consent Form.

**Exclusion criteria:**

1. subjects who received chemotherapy, radiotherapy, biological therapy, endocrine therapy, targeted therapy, immunotherapy or other antitumor therapies within 4 weeks prior to the first administration of the study drug, or participated in another clinical trial within 4 weeks or 5 half-lives of the investigational drug (whichever was shorter);

2. subjects with known clinical symptoms of central nervous system metastases or meningeal metastases, or uncontrolled central nervous system metastases or meningeal metastases indicated by other evidence, and deemed by the investigators to be unsuitable for enrollment;

3. subjects with the adverse reactions from the previous antitumor therapies that had not yet recovered to CTCAE 5.0 Grade ≤ 1 (except for toxicities such as alopecia deemed non-risky by the investigators);

4. subjects who participated in another clinical trial concurrently, unless it was observational (non-interventional) or in the follow-up period of an interventional trial;

5. subjects who had undergone major surgery or invasive intervention within 28 days prior to the first dose;

6. subjects who took any Chinese herbal medicine or Chinese patent medicine with antitumor activity approved by the China National Medical Products Administration (NMPA) within 14 days prior to the first dose (regardless of the type of cancer).

## V. Results

Arterial infusion of paclitaxel cationic liposome injection exhibited favorable efficacy on treatment of advanced solid tumors (for example, gastric cancer, pancreatic cancer, and colorectal cancer with liver metastases), could improve the efficacy and reduce the incidence of adverse reactions, and had a good prospect for clinical application.

Although the foregoing descriptions are merely the specific embodiments of the present disclosure, the scope of protection of the present disclosure is not limited thereto. Any variations or substitutions within the technical scope of the present disclosure, which are readily conceivable to a technician familiar with this technical field, should be encompassed in the scope of protection of the present disclosure. Therefore, the scope of protection of the present disclosure should be subject to the scope of protection of the claims.

## Claims

1. Use of a paclitaxel cationic liposome in preparation of a medicament for treating advanced solid tumors.

2. Use of a paclitaxel cationic liposome and a systemic therapeutic drug in preparation of a medicament for treating advanced solid tumors, preferably the systemic therapeutic drug being capecitabine and oxaliplatin, or cisplatin, or gemcitabine, or capecitabine.

3. A method for treating advanced solid tumors, wherein a therapeutically effective amount of a paclitaxel cationic liposome is administered to a patient with advanced solid tumors.

4. A method for treating advanced solid tumors, wherein a therapeutically effective amount of a paclitaxel cationic liposome and a systemic therapeutic drug are administered to a patient with advanced solid tumors, preferably a therapeutically effective amount of the paclitaxel cationic liposome, capecitabine, and oxaliplatin are adminsitered to the patient with the advanced solid tumors, or preferably the paclitaxel cationic liposome is administered via arterial infusion.

5. A method for improving the efficacy of a systemic therapeutic drug on tumors, wherein a therapeutically effective amount of a paclitaxel cationic liposome is further administered to a patient in addition to the systemic therapeutic drug, preferably the systemic therapeutic drug is capecitabine and oxaliplatin, or cisplatin, or gemcitabine, or capecitabine.

6. A method for improving the efficacy of a combination of capecitabine and oxaliplatin on advanced solid tumors including advanced gastric cancer, colorectal cancer, and pancreatic cancer, wherein a therapeutically effective amount of a paclitaxel cationic liposome is further administered to a patient in addition to capecitabine and oxaliplatin.

7. The use according to claim 1 or 2 or the method according to any one of claims 3 to 6, wherein the advanced solid tumors comprises gastrointestinal tumors including gastric cancer, esophageal cancer, pancreatic cancer, colorectal cancer, cholangiocarcinoma, and liver cancer; lung cancer; gynecological tumors including ovarian cancer, endometrial cancer, and cervical cancer; prostate cancer; bladder cancer; and solid tumor with liver metastases.

8. The use according to claim 1 or 2 or the method according to any one of claims 3 to 6, wherein the advanced solid tumors comprises gastric cancer, pancreatic cancer, or colorectal cancer with liver metastases, preferably, the advanced solid tumors comprises treatment-naive liver metastases including gastric cancer with liver metastases, colorectal cancer with liver metastases, or pancreatic cancer with liver metastases.

9. The method according to any one of claims 3 to 8, wherein on a paclitaxel basis, the therapeutically effective amount of the paclitaxel cationic liposome is 5 to 100 mg/m$^2$, preferably 5 to 80 mg/m$^2$, further preferably 11 to 55 mg/m$^2$, or yet further preferably 24 to 70 mg/m$^2$, or any value within the range, including 11 mg/m$^2$, 22 mg/m$^2$, 24 mg/m$^2$, 33 mg/m$^2$, 36 mg/m$^2$, 44 mg/m$^2$, 48 mg/m$^2$, 55 mg/m$^2$, 60 mg/m$^2$, and 70 mg/m$^2$, preferably the paclitaxel cationic liposome is administered via arterial infusion, preferably a dosing cycle is once every 3 weeks for 6 to 8 cycles.

10. The use according to claim 1 or 2 or the method according to any one of claims 3 to 8, wherein the paclitaxel cationic liposome contains: paclitaxel, 1,2-dioleoyl-sn-glycero-3-phosphocholine (DOPC), 1,2-dioleoyl-3-trimethylammonium-propane chloride (DOTAP), and trehalose; preferably each preparation of the paclitaxel cationic liposome contains 5 to 10 mg of paclitaxel, 72 to 144 mg of 1,2-dioleoyl-sn-glycero-3-phosphocholine, 68 to 136 mg of 1,2-dioleoyl-3-trimethylammonium-propane chloride, and 1500 to 2500 mg of trehalose.

**FIG. 1**

## INTERNATIONAL SEARCH REPORT

| | |
|---|---|
| International application No. | |
| | **PCT/CN2024/074604** |

**A. CLASSIFICATION OF SUBJECT MATTER**

A61K 9/127(2006.01)i; A61K31/337(2006.01)i; A61K31/7068(2006.01)i; A61K33/24(2019.01)i; A61K31/282(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext; 读秀, DUXIU: 紫杉醇, 脂质体, 阳离子, 癌, 肿瘤, 卡培他滨, 奥沙利铂, 顺铂, 吉西他滨, Paclitaxel, Taxol, DOTAP, Liposome, cation, carcinoma, cancer, tumor, capecitabine, oxaliplatin, cisplatin, gemcitabine

**C. DOCUMENTS CONSIDERED TO BE RELEVANT**

| Category* | Citation of document, with indication, where appropriate, of the relevant passages | Relevant to claim No. |
|---|---|---|
| X | US 2009047337 A1 (MESCHEDER AXEL et al.) 19 February 2009 (2009-02-19)<br>description, paragraphs [0028]-[0036], [0057], [0067], [0073] and [0089] | 1, 2, 7 (in part), 8 (in part), 10 (in part) |
| X | US 2008063699 A1 (TEIFEL MICHAEL et al.) 13 March 2008 (2008-03-13)<br>claims 1-35 | 1, 2, 7 (in part), 8 (in part), 10 (in part) |
| X | US 2019015332 A1 (SYNCORE BIOTECHNOLOGY CO., LTD. et al.) 17 January 2019 (2019-01-17)<br>claims 1-27 | 1, 2, 7 (in part), 8 (in part), 10 (in part) |
| X | US 2019380994 A1 (SYNCORE BIOTECHNOLOGY CO., LTD.) 19 December 2019 (2019-12-19)<br>description, paragraphs [0094]-[0143] | 1, 2, 7 (in part), 8 (in part), 10 (in part) |
| X | Amr S. Abu Lila et al. "Sequential Administration with Oxaliplatin-containing PEG-coated Cationic Liposomes Promotes a Significant Delivery of Subsequent Dose into Murine Solid Tumor"<br>*Journal of Controlled Release*, Vol. vol. 142, 25 October 2009 (2009-10-25), 167-173<br>abstract | 1, 2, 7 (in part), 8 (in part), 10 (in part) |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 April 2024** | **03 May 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/074604** |

## C. DOCUMENTS CONSIDERED TO BE RELEVANT

| Category* | Citation of document, with indication, where appropriate, of the relevant passages | Relevant to claim No. |
| --- | --- | --- |
| X | Martin E. Eichhorn et al. "Vascular Targeting by EndoTAG TM-1 Enhances Therapeutic Efficacy of Conventional Chemotherapy in Lung and Pancreatic Cancer" *International Journal of Cancer*, Vol. vol. 126, 20 August 2009 (2009-08-20), pp. 1235-1245 abstract, and page 1236, left-hand column, last paragraph | 1, 2, 7 (in part), 8 (in part), 10 (in part) |
| Y | 刘君等 (LIU, Jun et al.). "紫杉醇脂质体联合奥沙利铂和卡培他滨一线治疗晚期胃癌临床观察 (Clinical Observation of Paclitaxel Liposome Combined with Oxaliplatin and Capecitabine in the First-line Treatment of Advanced Gastric Cancer)" *现代中西医结合杂志 (Modern Journal of Integrated Traditional Chinese and Western Medicine)*, Vol. 20, No. (3), 31 January 2011 (2011-01-31), pp. 268-273 abstract | 6 |
| Y | US 2009047337 A1 (MESCHEDER AXEL et al.) 19 February 2009 (2009-02-19) description, paragraphs [0028]-[0036], [0057], [0067], [0073] and [0089] | 6 |

Form PCT/ISA/210 (second sheet) (July 2022)

**INTERNATIONAL SEARCH REPORT**

International application No.

**PCT/CN2024/074604**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **3-6, 7 (in part), 8 (in part), 9, 10 (in part)**
    because they relate to subject matter not required to be searched by this Authority, namely:

    Claims 3-6 and 9, and claims 7, 8 and 10, when referring to said claims, set forth a disease treatment method that is practiced on a living human or animal body for the direct purpose of disease treatment, which falls within methods for treatment of the human or animal body by therapy, and falls within the cases set out in PCT Rule 39.1(iv) for which an international search is not required.

    The search and written opinion with regard to claim 6 are made on the basis of the following reasonable expectation:

    Claim 6: the use of capecitabine, oxaliplatin and paclitaxel cationic liposome in the preparation of a drug for treating advanced solid tumors, wherein the advanced solid tumors comprise advanced gastric cancer, colorectal cancer and pancreatic cancer.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/074604**

| Patent document cited in search report | | | Publication date (day/month/year) | Patent family member(s) | | | Publication date (day/month/year) |
|---|---|---|---|---|---|---|---|
| US | 2009047337 | A1 | 19 February 2009 | US | 9233094 | B2 | 12 January 2016 |
| | | | | EP | 1896007 | A2 | 12 March 2008 |
| | | | | EP | 1896007 | B1 | 19 March 2014 |
| | | | | US | 2016081929 | A1 | 24 March 2016 |
| | | | | US | 9827196 | B2 | 28 November 2017 |
| | | | | AU | 2006243337 | A1 | 09 November 2006 |
| | | | | AU | 2006243337 | B2 | 29 September 2011 |
| | | | | JP | 2008540364 | A | 20 November 2008 |
| | | | | JP | 5700909 | B2 | 15 April 2015 |
| | | | | WO | 2006117220 | A2 | 09 November 2006 |
| | | | | WO | 2006117220 | A3 | 11 January 2007 |
| | | | | CA | 2601067 | A1 | 09 November 2006 |
| US | 2008063699 | A1 | 13 March 2008 | CA | 2542217 | A1 | 06 May 2005 |
| | | | | CA | 2542217 | C | 27 March 2018 |
| | | | | HUE | 028648 | T2 | 28 December 2016 |
| | | | | PT | 2286794 | T | 13 July 2016 |
| | | | | ES | 2574231 | T3 | 16 June 2016 |
| | | | | EP | 2286794 | A1 | 23 February 2011 |
| | | | | EP | 2286794 | B1 | 30 March 2016 |
| | | | | EP | 2286794 | B8 | 01 June 2016 |
| | | | | WO | 2005039533 | A1 | 06 May 2005 |
| | | | | JP | 2012229255 | A | 22 November 2012 |
| | | | | JP | 2007508353 | A | 05 April 2007 |
| | | | | JP | 5645340 | B2 | 24 December 2014 |
| | | | | DK | 2286794 | T3 | 25 July 2016 |
| | | | | PL | 2286794 | T3 | 31 October 2016 |
| | | | | AU | 2004283464 | A1 | 06 May 2005 |
| | | | | AU | 2004283464 | B2 | 17 March 2011 |
| | | | | AU | 2004283464 | B8 | 14 April 2011 |
| | | | | EP | 1673069 | A1 | 28 June 2006 |
| | | | | EP | 1673069 | B1 | 12 October 2016 |
| US | 2019015332 | A1 | 17 January 2019 | KR | 20180093077 | A | 20 August 2018 |
| | | | | JP | 2019505582 | A | 28 February 2019 |
| | | | | JP | 6912496 | B2 | 04 August 2021 |
| | | | | MX | 2018008196 | A | 28 August 2018 |
| | | | | TW | 201722423 | A | 01 July 2017 |
| | | | | TWI | 760319 | B | 11 April 2022 |
| | | | | ES | 2902576 | T3 | 29 March 2022 |
| | | | | AU | 2016383697 | A1 | 19 July 2018 |
| | | | | AU | 2016383697 | B2 | 18 November 2021 |
| | | | | EP | 3397244 | A1 | 07 November 2018 |
| | | | | EP | 3397244 | A4 | 04 September 2019 |
| | | | | EP | 3397244 | B1 | 06 October 2021 |
| | | | | US | 2022313608 | A1 | 06 October 2022 |
| | | | | WO | 2017115301 | A1 | 06 July 2017 |
| | | | | CA | 3009608 | A1 | 06 July 2017 |
| US | 2019380994 | A1 | 19 December 2019 | MX | 2022005350 | A | 02 June 2022 |
| | | | | BR | 112019013983 | A2 | 28 April 2020 |
| | | | | MX | 2019008132 | A | 13 September 2019 |
| | | | | RU | 2019124441 | A | 05 February 2021 |

Form PCT/ISA/210 (patent family annex) (July 2022)

## INTERNATIONAL SEARCH REPORT
### Information on patent family members

International application No.

**PCT/CN2024/074604**

| Patent document cited in search report | Publication date (day/month/year) | Patent family member(s) | | | Publication date (day/month/year) |
|---|---|---|---|---|---|
| | | RU | 2019124441 | A3 | 21 April 2021 |
| | | EP | 3565530 | A1 | 13 November 2019 |
| | | EP | 3565530 | A4 | 01 July 2020 |
| | | AU | 2018205544 | A1 | 27 June 2019 |
| | | AU | 2018205544 | B2 | 29 February 2024 |
| | | TW | 201825087 | A | 16 July 2018 |
| | | WO | 2018127082 | A1 | 12 July 2018 |
| | | CA | 3049183 | A1 | 12 July 2018 |
| | | JP | 2022180464 | A | 06 December 2022 |
| | | KR | 20190103304 | A | 04 September 2019 |
| | | JP | 2020504138 | A | 06 February 2020 |
| | | IL | 267613 | A | 29 August 2019 |
| | | US | 2023035723 | A1 | 02 February 2023 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310046953
- US 7794747 B

**Non-patent literature cited in the description**

- *The CSCO Guidelines for Liver Cancer*, 2022
- Chinese tumor intervention expert consensus on the application principles of transcatheter arterial infusion chemotherapy. *J Intervent Radiol*, November 2017, vol. 26 (11)
- **CASSIDY J** ; **CLARKE S** ; **DÍAZ-RUBIO E** ; **SCHEITHAUER W** ; **FIGER A** ; **WONG R** ; **KOSKI S** ; **LICHINITSER M** ; **YANG TS** ; **RIVERA F**. Randomized phase III study of capecitabine plus oxaliplatin compared with fluorouracil/folinic acid plus oxaliplatin as first-line therapy for metastatic colorectal cancer. *J Clin Oncol.*, 20 April 2008, vol. 26 (12), 2006-12
- **S. STRIETH** ; **M.E. EICHHORN** ; **B. SAUER et al.** Neovasculartargeting chemotherapy: encapsulation of paclitaxel in cationic liposomes impairs functional tumor microvasculature. *Int. J. Cancer*, 2004, vol. 110, 117-124
- **MA WW** ; **HIDALGO M**. The winning formulation: the development of paclitaxel in pancreatic cancer. *Clin Cancer Res.*, 05 August 2013, vol. 19 (20), 5572-9
- **CAMACHO LH** ; **KURZROCK R** ; **CHEUNG A** ; **BARBER DF** ; **GUPTA S** ; **MADOFF DC** ; **WALLACE MJ** ; **KIM EE** ; **CURLEY SA** ; **HORTOBAGYI GN**. Pilot study of regional, hepatic intra-arterial paclitaxel in patients with breast carcinoma metastatic to the liver. *Cancer*, 01 June 2007, vol. 109 (11), 2190-6